**Europäisches Patentamt**

**European Patent Office**

**Office européen des brevets**

(11) Veröffentlichungsnummer: **0 044 268**
**B1**

# (12) EUROPÄISCHE PATENTSCHRIFT

(45) Veröffentlichungstag der Patentschrift:
**23.11.83**

(21) Anmeldenummer: **81730065.0**

(22) Anmeldetag: **09.07.81**

(51) Int. Cl.³: **A 61 N 1/04**

(30) Priorität: **16.07.80 DE 3027383**

(43) Veröffentlichungstag der Anmeldung:
**20.01.82 Patentblatt 82/3**

(45) Bekanntmachung des Hinweises auf die Patenterteilung:
**23.11.83 Patentblatt 83/47**

(84) Benannte Vertragsstaaten:
**AT CH DE FR GB IT LI NL SE**

(56) Entgegenhaltungen:
**EP - A - 0 003 948**
**FR - A - 2 319 384**

**BIOMEDIZINISCHE TECHNIK, Band 24, Heft 1/2,
Januar/Februar 1979, BERLIN (DE) H.J. BISPING:
"Übersicht über verankerbare transvenöse
Schrittmachersonden", Seiten 16-27**

(73) Patentinhaber: **BIOTRONIK Mess- und Therapiegeräte
GmbH & Co Ingenieurbüro Berlin, Sieversufer 8,
D-1000 Berlin 47 (DE)**

(72) Erfinder: **Theisen, Peter, Dipl.-Ing.,
Onkel-Herse-Strasse 26, D-1000 Berlin 47 (DE)**
Erfinder: **Riechert, Klaus-Dieter, Leydenallee 38,
D-1000 Berlin 41 (DE)**

(74) Vertreter: **Christiansen, Henning, Dipl.-Ing., Unter den
Eichen 108a, D-1000 Berlin 45 (DE)**

(54) **Transvenöse Herzschrittmacherelektrode.**

Transvenöse Herzschrittmacherelektrode

Die Erfindung betrifft eine transvenöse Herzschrittmacherelektrode der im Oberbegriff des Anspruchs 1 angegebenen Art.

Eine derartige Herzschrittmacherelektrode ist beispielsweise aus der DE-OS 21 33 304 bekannt.

Diese Elektrode ist mit dem Nachteil behaftet, dass beim Einführen der Elektrode unter Röntgenkontrolle vom implantierenden Chirurgen im Röntgenbild nicht mit ausreichender Deutlichkeit erkannt werden kann, ob sich die Befestigungsmittel im ein- oder ausgefahrenen Zustand befinden.

Im Interesse eines komplikationsfreien Eingriffes ist es wünschenswert, dass der implantierende Chirurg jederzeit ohne zusätzliche Hilfsmittel oder Betätigungen feststellen kann, welche Position die Befestigungsmittel einnehmen, was insbesondere dann von Bedeutung ist, wenn die Elektrode aus einer ursprünglich für die Fixierung vorgesehenen Position wieder entfernt und in einen anderen Bereich des Herzens verschoben werden muss.

Die Möglichkeit der Röntgenkontrolle bei der Implantation von Herzschrittmacherelektroden ist in der US-PS 3 754 555 (Spalte 2, Zeilen 28 bis 30) erwähnt. Auch bei der aus dieser Druckschrift bekannten Elektrode erweist es sich als nachteilig, dass die Befestigungsmittel zum Fixieren der Elektrode in der Herzkammer aus Metall vorgegebener Mindeststärke gefertigt sein müssen, damit sie im Röntgenbild deutlich sichtbar werden.

Es ist ferner bekannt, dass bei einem innerhalb eines Hohlzylinders beweglichen Kolben, der vorzugsweise in ausgefahrener Stellung der Befestigungsmittel eine Dichtung gegen in das Innere der Elektrode eindringende Körperflüssigkeit bildet, der Kolben als in der Regel massiv ausgebildeter Metallkörper am besten in der Lage ist, auf dem Röntgenbild eine deutlich sichtbare Positionsmarkierung abzugeben, vgl. EP-A-0 003 948. Bei dieser Konstruktion sind jedoch keine Massnahmen getroffen, die eine Kenntnis der Position des Befestigungsmittels ermöglichen.

Der im Anspruch 1 angegebenen Erfindung liegt die Aufgabe zugrunde, eine Herzschrittmacherelektrode der eingangs genannten Art zu geben, welche von den erwähnten Nachteilen frei ist und die dem Arzt jederzeit eine genaue Kenntnis der Position der Befestigungsmittel ermöglicht.

Diese Aufgabe wird mit den im kennzeichnenden Teil des Anspruchs 1 angegebenen konstruktiven Massnahmen gelöst.

Dadurch, dass die Bereiche verringerter Materialstärke im – in bezug auf die Einführungsrichtung der Elektrode – rückwärtigen Bereich des Kolbenweges angeordnet sind, sind diese bei ausgefahrenen Befestigungselementen (Kolben in der vorderen Position) für im wesentlichen seitlich einfallende Röntgenstrahlung frei, d.h. auch Teile der Befestigungsmittel selbst blockieren den Weg der Röntgenstrahlung nicht. Die Bereiche mit vergrösserter Durchlässigkeit für Röntgenstrahlung können dabei als Schwächungen der Wandstärke des Hohlzylinders bzw. als Durchbrüche vorgesehen sein. Die beim Vorsehen von Durchbrüchen notwendigerweise hervorgerufene Abweichung der Form der Innenseite des Hohlzylinders von der geometrischen Zylinderform stellt keinen Nachteil für die Dichtwirkung des Kolbens dar, da diese erst bei ausgefahrenem Befestigungselement, d.h. vorangeschobenem Kolben, vollständig vorhanden sein sollte.

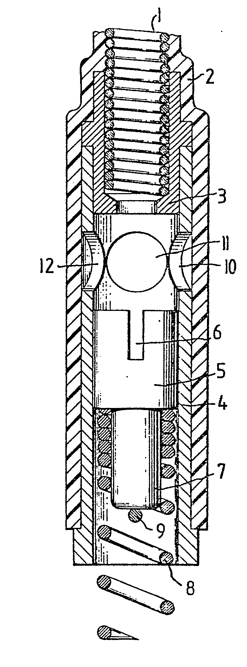

Weitere vorteilhafte Weiterbildungen der Erfindung sind in den Unteransprüchen angegeben, bzw. werden nachfolgend anhand eines in der Zeichnung dargestellten Ausführungsbeispiels näher beschrieben.

Die einzige Figur zeigt das zur Befestigung im Herzen bestimmte Ende der erfindungsgemässen Herzschrittmacherelektrode.

Bei dem dargestellten Ausführungsbeispiel dient eine Zuleitungswendel 1, die von einem Mantel 2 aus Silikonkautschuk umgeben ist, zur elektrischen Verbindung zwischen Schrittmacher-Aus- bzw. -Eingang und dem zu stimulierenden Bereich des Herzgewebes. Die Zuleitungswendel 1 ist dabei verkürzt dargestellt und die Mittel zur elektrischen Kontaktierung des Schrittmacheranschlusses wurden aus Gründen der Übersichtlichkeit weggelassen.

Das herznahe Ende der Zuleitungswendel 1 endet in einer Hülse 3, welche ihrerseits von einem Hohlzylinder 4 umgeben ist. Auch dieser Hohlzylinder 4 ist in seinem der Zuleitungswendel 1 benachbarten Bereich weitgehend von dem Mantel 2 aus Silikonkautschuk umgeben, der die nicht zur Stimulation bzw. zum Erfassen von Herzsignalen beitragenden Bereiche der Elektrode gegenüber dem Herzgewebe elektrisch isoliert.

Innerhalb des Hohlzylinders 4 ist ein in Längsrichtung verschiebbarer Kolben 5 angeordnet, welcher in seinem rückwärtigen Bereich eine Nut 6 aufweist, in die ein mit einem schraubenzieherartigen Ende versehener – ebenfalls nicht dargestellter – Führungsdraht eingreifen kann. Das vordere Ende des Kolbens 5 weist einen Ansatz 7 auf, welcher den Träger einer korkenzieherförmigen Schraubwendel 8 bildet, die zur Fixierung der Elektrode im Herzgewebe dient.

Mittels der Nut 6 und der Gewindewirkung eines im Hohlzylinder 4 fest angebrachten Querstiftes 9 ist die Schraubwendel 8 durch Ausführung einer Drehbewegung ein- bzw. ausfahrbar.

Zum Ausfahren wird, wenn das herznahe Ende der Elektrode den vorgesehenen Fixierungsort erreicht hat, über den mit schraubenzieherförmigen Ansatz versehenen Führungsdraht ein Drehmoment auf die Nut 6 ausgeübt und damit der Kolben samt der Schraubwendel in die ausgefahrene Position geführt. Dabei bewegt sich der Kolben 5 aus einer Position, in der er in dem Hohlzylinder vorgesehene Öffnungen 10, 11 und 12 verdeckt, in die in der Zeichnung dargestellte Position, in der eine Durchstrahlung des Hohlzylinders aus im wesentlichen seitlicher Richtung nicht durch den Kolben behindert wird. Damit ist es dem implantierenden Arzt ohne weiteres möglich, bei Röntgenkontrolle die Position des Kolbens in bezug auf den Hohlzylinder – und damit diejenige der Befestigungsmittel (Schraubwendel 8) – zu verfolgen und ist nicht darauf angewiesen, diese beispielsweise über den Führungsdraht zu ertasten oder durch Auszählen der Anzahl der ausgeführten Umdrehungen des Führungsdrahtes im Gedächtnis zu behalten.

Die Anordnung der Öffnungen 10 bis 12 ist so gewählt, dass bei im wesentlichen seitlich einfallender Röntgenstrahlung unabhängig von der Bestrahlungsrichtung flächenmässig im Röntgenbild deutlich in Erscheinung tretende Bereiche vorhanden sind, welche bei ausgefahrenem Befestigungselement freigegeben sind. Damit ist es dem implantierenden Chirurgen möglich, unabhängig von der axialen Richtung der Elektrode die Position des Hohlzylinders 4 zu erkennen. Dabei stellt es keinen Nachteil dar, dass die Röntgenstrahlung im wesentlichen seitlich einfallen muss, um die Öffnungen zu treffen, da das Elektrodenende in seiner Position zum Fixieren Ventrikel im wesentlichen parallel zur Längsrichtung des Körpers des Patienten verläuft, so dass die Gewähr gegeben ist, dass hier die Position des Kolbens 5 in bezug auf die Öffnungen deutlich sichtbar in Erscheinung tritt. Auf dem Röntgenschirm wird der Arzt sich dabei entweder an der bei in seiner ausgefahrenen Stellung befindlichen Kolben sichtbar werdenden Struktur der Öffnungen (runde Löcher bei dem dargestellten Ausführungsbeispiel) in der Abgrenzung zu den ungeschwächten Materialbereichen des Hohlzylinders oder aber an der Grenze seiner geschwächten Bereiche in bezug auf die Hinterkante des Kolbens orientieren.

Die Öffnungen sind bei dem dargestellten Ausführungsbeispiel so angeordnet, dass seitlich einfallendes Röntgenlicht bei in ihrer ausgefahrenen Position befindlichen Einschraubwendel nacheinander durch jeweils zwei einander gegenüber angeordnete Öffnungen (wie beispielsweise durch die Öffnungen 10 und 12) fällt, so dass hier eine minimale Behinderung der Strahlen eintritt. Die durchstrahlten Öffnungen werden in diesem Fall also auf dem Röntgenschrim besonders deutlich wiedergegeben, so dass der Unterschied zu dem Zustand, in dem ein Verdecken durch den eingefahrenen Kolben 5 eintritt, auffallend ist. Die als Durchbrüche ausgebildeten Öffnungen werden von dem den Hohlzylinder 4 umgebenden Mantel abgedeckt, so dass keine Körperflüssigkeit in das Innere der Elektrode gelangen kann.

Um zu erreichen, dass auch in einem Winkel zur radialen Richtung des Hohlzylinders 4 einfallendes Röntgenlicht durch die Öffnungen 10 bis 11 fällt, ist es vorteilhaft, wenn deren Erstreckung in Längsrichtung des Hohlzylinders 4 möglichst gross ist, so dass auch noch die Gewähr gegeben ist, dass das von schräg seitlich einfallende Röntgenlicht zwei der Öffnungen 10 bis 12 nacheinander passiert. Andererseits sind die Abmessungen der Öffnungen in Umfangsrichtung bevorzugt so zu wählen, dass diese grösser sind als der Durchmesser üblicherweise verwendeter Führungsdrähte, so dass, auch wenn ein derartiger Führungsdraht bis in die Nut 6 des Kolbens 5 vorangeschoben ist, eine Beurteilung der Position der Zuleitungswendel leicht möglich ist.

## Patentansprüche

1. Transvenöse Herzschrittmacherelektrode mit einem an einem innerhalb eines Hohlzylinders verschieblichen Kolben angebrachten Befestigungselement zum Fixieren der Elektrode im Herzen, wobei das Befestigungselement zum Einführen der Elektrode in eine geschützte Position innerhalb des Hohlzylinders zurückschiebbar ist, dadurch gekennzeichnet, dass nur bei eingefahrenem Befestigungselement (8) dem Kolben (5) mindestens ein Wandungsbereich des Hohlzylinders (4) benachbart ist, der eine mindestens derartig verringerte Materialstärke aufweist, dass bei Röntgendurchstrahlung die Grenze des Bereichs zu den Gebieten mit ungeschwächter Materialstärke bzw. zum distalen Ende des Kolbens deutlich sichtbar in Erscheinung tritt, wenn der Kolben sich in der ausgefahrenen Position des Befestigungselements befindet.

2. Herzschrittmacherelektrode nach Anspruch 1, dadurch gekennzeichnet, dass der Bereich mit verringerter Materialstärke einen Durchbruch (10, 11, 12) bildet.

3. Herzschrittmacherelektrode nach Anspruch 2, dadurch gekennzeichnet, dass der den Durchbruch enthaltende Bereich des Hohlzylinders (4) von einem Mantel (2) aus Silikonkautschuk umgeben ist.

4. Herzschrittmacherelektrode nach einem der vorangehenden Ansprüche, dadurch gekennzeichnet, dass der Bereich verringerter Materialstärke derart ausgebildet ist, dass bei im wesentlichen seitlich auf den Hohlzylinder (4) einfallenden Röntgenlicht unabhängig von der Einfallsrichtung jeweils ein Bezirk von bei Röntgenkontrolle deutlich sichtbar in Erscheinung tretender Grösse durchstrahlt wird.

5. Herzschrittmacherelektrode nach einem der Ansprüche 2 bis 4, dadurch gekennzeichnet, dass mehrere auf dem Umfang verteilte Bereiche verringerter Materialstärke vorgesehen sind, wobei das zwischen benachbarten Bereichen verbleibende Material eine für die Festigkeit des Hohlzylinders (4) ausreichenden Querschnitt aufweist.

6. Herzschrittmacherelektrode nach Anspruch 5, dadurch gekennzeichnet, dass jeweils zwei Bereiche verringerter Materialstärke einander gegenüber angeordnet sind.

7. Herzschrittmacherelektrode nach Anspruch 6, dadurch gekennzeichnet, dass die Bereiche verringerter Materialstärke eine sich bevorzugt in Längsrichtung des Hohlzylinders (4) erstreckende Gestalt aufweisen.

8. Herzschrittmacherelektrode nach einem der vorangehenden Ansprüche, dadurch gekennzeichnet, dass die Abmessungen der Bereiche in Umfangsrichtung des Hohlzylinders grösser ist als der Durchmesser des Führungsdrahts.

## Revendications

1. Electrode transveineuse pour stimulateur cardiaque, comportant, pour fixation de l'électrode dans le cœur, un élément de fixation rapporté sur un piston qui peut coulisser à l'intérieur d'un cylindre creux, cet élément de fixation pouvant, pour l'introduction de l'électrode, être ramené en position protégée à l'intérieur du cylindre creux, caractérisé en ce que ce n'est que lorsque l'élément de fixation (8) est en position de rétraction, qu'au moins une zone de paroi du cylindre creux (4) est voisine du piston (5), zone de paroi qui présente une épaisseur de matériau au moins réduite de façon telle que, dans le cas du passage d'un faisceau de rayons X, la limite de cette zone avec les zones dont l'épaisseur de matériau n'est pas affaiblie ou avec l'extrémité éloignée du piston apparaît nettement visible si le piston se trouve en position en extension de l'élément de fixation.

2. Electrode pour stimulateur cardiaque selon la revendication 1, caractérisée en ce que la zone à épaisseur de matériau réduite forme une découpe (10, 11, 12).

3. Electrode pour stimulateur cardiaque selon la revendication 2, caractérisée en ce que la zone du cylindre creux (4) qui contient la découpe est entourée d'une enveloppe (2) en caoutchouc silicone.

4. Electrode pour stimulateur cardiaque selon l'une quelconque des revendications précédentes, caractérisée en ce que la zone d'épaisseur de matériau réduite a une forme telle que dans le cas d'un faisceau de rayons X tombant essentiellement latéralement sur le cylindre creux (4), quelle que soit la direction incidente, un domaine d'une taille donnant une image nettement visible lors du trôle radio soit chaque fois traversé par le faisceau.

5. Electrode pour stimulateur cardiaque selon l'une des revendications 2 à 4, caractérisée en ce qu'il est prévu plusieurs zones réparties sur la périphérie, d'épaisseur de matériau réduite, le matériau qui subsiste entre les zones voisines présentant une section suffisante pour la résistance du cylindre creux (4).

6. Electrode pour stimulateur cardiaque selon la revendication 5, caractérisée en ce que deux zones d'épaisseur de matériau réduite sont respectivement disposées l'une en face de l'autre.

7. Electrode pour stimulateur cardiaque selon la revendication 6, caractérisée en ce que les zones d'épaisseur de matériau réduite présentent une forme qui s'étend de préférence en direction longitudinale du cylindre creux (4).

8. Electrode pour stimulateur cardiaque selon l'une des revendications précédentes, caractérisée en ce que les dimensions des zones selon la direction périphérique du cylindre creux sont supérieures à celle du diamètre du fil de guidage.

## Claims

1. Transvenous heart pace-maker electrode with an attachment element mounted on a plunger displaceable within a hollow cylinder for fixing the electrode in the heart, the attachment element being capable of being pushed back into a protected position within the hollow cylinder for inserting the electrode, characterised in that only when the attachment element (8) is retracted is there adjacent to the plunger (5) a wall region of the hollow cylinder (4) which has a material thickness that is at least so reduced that, upon X-ray irradiation, the junction of the region with the zones with unimpaired material strength or with the distal end of the plunger is clearly apparent when the plunger is situated in the extended position of the attachment element.

2. Heart pace-maker electrode according to claim 1, characterised in that the region with reduced material thickness forms an opening (10, 11, 12).

3. Heart pace-maker electrode according to claim 2, characterised in that the region of the hollow cylinder (4) containing the opening is surrounded by a sheath of silicone rubber.

4. Heart pace-maker electrode according to any one of the proceding claims characterised in that the region of reduced material thickness is so formed that, upon X-rays falling essentially laterally upon the hollow cylinder, there is irradiated in each case independently of the direction of incidence an area with a size which is clearly apparent upon X-ray inspection.

5. Heart pace-maker electrode according to any one of claims 2 to 4, characterised in that a plurality of distributed regions of reduced material thickness are provided at the periphery, the material remaining between adjacent regions having a cross-section sufficient for the strength of the hollow cylinder (4).

6. Heart pace-maker electrode according to claim 5, characterised in that in each case two regions of reduced material thickness are arranged opposite each other.

7. Heart pace-maker electrode according to claim 6, characterised in that the regions of reduced material thickness have a shape which preferably extends in the longitudinal direction of the hollow cylinder (4).

8. Heart pace-maker electrode according to any one of the preceding claims characterised in that the dimensions of the regions in the peripheral direction of the hollow cylinder is greater than the diameter of the guide wire.